(19) Europäisches Patentamt
European Patent Office
Office européen des brevets

(11) **EP 2 554 111 A1**

(12) **EUROPEAN PATENT APPLICATION**
published in accordance with Art. 153(4) EPC

(43) Date of publication:
**06.02.2013 Bulletin 2013/06**

(51) Int Cl.:
**A61B 5/02** (2006.01)

(21) Application number: **11762493.2**

(22) Date of filing: **07.03.2011**

(86) International application number:
**PCT/JP2011/055226**

(87) International publication number:
**WO 2011/122253 (06.10.2011 Gazette 2011/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2010 JP 2010078682**

(71) Applicant: **Sharp Kabushiki Kaisha
Osaka-shi, Osaka 545-8522 (JP)**

(72) Inventor: **HORI, Atsushi
Osaka-shi, Osaka 545-8522 (JP)**

(74) Representative: **Treeby, Philip David William et al
R.G.C. Jenkins & Co
26 Caxton Street
London
Greater London SW1H 0RJ (GB)**

(54) **PULSE WAVE VELOCITY MEASUREMENT DEVICE, PULSE WAVE VELOCITY MEASUREMENT METHOD AND PULSE WAVE VELOCITY MEASUREMENT PROGRAM**

(57) A pulse wave velocity measurement device comprises a pulse wave detection unit (110) for detecting a pulse wave in a living body, a pulse wave velocity calculation unit (120) for calculating a pulse wave velocity on basis of the pulse wave detected by the pulse wave detection unit (110), and a pulse wave velocity correction unit (130) for correcting the pulse wave velocity calculated by the pulse wave velocity calculation unit (120) so as to eliminate an increment in the pulse wave velocity that results from influence of hydrostatic pressures caused according to a position of the living body. Thus, the pulse wave velocity measurement device can be provided that are capable of accurate measurement of the pulse wave velocity without being influenced by the hydrostatic pressures caused according to the position of the living body.

Fig.1

**Description**

TECHNICAL FIELD

The present invention relates to a pulse wave velocity measurement device and particularly to a pulse wave velocity measurement device, a pulse wave velocity measurement method, and a pulse wave velocity measurement program that are for measuring pulse waves of a living body and thereby calculating velocity at which the pulse waves are propagated.

BACKGROUND ART

It has been known that pulse waves have various important information for grasp on a state of a circulatory system of a living body. In particular, velocity and time for propagation of a pulse wave between two sites in a living body are living-body indices that have drawn attention from the medical work front because a possibility has been pointed out that a state such as arteriosclerosis may be grasped by the indices, and are called Pulse Wave Velocity (PWV) and Pulse Transit Time (PTT), respectively, or the like.

In general, two measurement points are required for PWV. It is known, however, that a pulse wave measured at any site on at living body is a resultant wave of an ejection wave from the heart and reflected waves reflected from various sites in the living body, and there is a possibility that separation thereof may make it possible to find the pulse wave velocity or the pulse transit time even if only one measurement point for the pulse wave exists.

Therefore, there have conventionally been proposed pulse wave velocity measurement devices that find a pulse wave velocity or a pulse transit time by separation of a pulse wave into an ejection wave and reflected waves on basis of pulse waves at one measurement point (see JP 2004-313468 A (Patent Literature 1) and JP 2003-010139 A (Patent Literature 2), for instance).

In the conventional pulse wave velocity measurement devices, the separation of the pulse wave into the ejection wave and the reflected waves is satisfactorily attained for pulse waves measured at various sites in a living body, on an assumption that a staple reflection point is in vicinity of the iliac arteries or the abdominal aorta.

The conventional pulse wave velocity measurement devices are not influenced by hydrostatic pressures because the measurement point and the heart are at the same height for reason that pulse waves at the measurement point are measured in a supine position and that pulse wave velocity is found on basis of time difference therebetween and distance.

In consideration of scenes of use by a user, however, it is inconvenient for the user to be required to lie supinely every time for the measurement of the pulse wave velocity. If the measurement can be performed in a sitting position or an upright position, for instance, the trouble to the user with the measurement can be reduced, and the pulse wave velocity can be measured any time and anywhere, and a wearable live body sensor can be provided.

For the separation of the pulse wave into the ejection wave and the reflected waves in the conventional pulse wave velocity measurement devices, it is assumed that the staple reflection point is in vicinity of the iliac arteries or the abdominal aorta. That is, path of the reflected waves from the abdominal aorta passes from the heart through the iliac arteries or the abdominal aorta and reaches the measurement point. When the measurement is performed in the sitting position or the upright position, for instance, the iliac arteries or the abdominal aorta is necessarily below the heart. Accordingly, pulse waves that pass through the path running from the heart through the iliac arteries or the abdominal aorta, being reflected, and reaching a peripheral artery are not influenced by hydrostatic pressures, and blood pressure values in the path are consequently increased. It has been known that there is a correlation between blood pressures and pulse wave velocities and that increase in the blood pressure results in increase in the pulse wave velocity. Therefore, the pulse wave velocity measured in the sitting position or the upright position is made higher than that measured in the supine position.

Thus the conventional pulse wave velocity measurement devices have a problem in that the devices cannot ensure accurate measurement because the pulse wave velocity is increased by hydrostatic pressure difference when the measurement is performed in the sitting position or the upright position, for instance, rather than in the supine position.

CITATION LIST

Patent Literature

PTL1: JP 2004-313468 A

PTL2: JP 2003-010139 A

SUMMARY OF INVENTION

Technical Problem

Therefore, an object of the invention is to provide a pulse wave velocity measurement device, a pulse wave velocity measurement method, and a pulse wave velocity measurement program by which accurate measurement of pulse wave velocity can be performed without being influenced by hydrostatic pressures caused according to a position of a living body.

Solution to Problem

In order to solve the problem, a pulse wave velocity measurement device according to the present invention comprises:

a pulse wave detection unit for detecting a pulse wave in a living body,
a pulse wave velocity calculation unit for calculating a pulse wave velocity on basis of the pulse wave detected by the pulse wave detection unit, and
a pulse wave velocity correction unit for correcting the pulse wave velocity calculated by the pulse wave velocity calculation unit so as to eliminate an increment in the pulse wave velocity that results from influence of hydrostatic pressures caused according to a position of the living body.

Herein, "living body" is not limited to a human body and may be other animals of which pulse waves of circulatory system can be measured.
According to the above configuration, the pulse wave velocity calculated by the pulse wave velocity calculation unit is corrected by the pulse wave velocity correction unit. This eliminates the increment in the pulse wave velocity that results from the influence of the hydrostatic pressures caused according to the position of the living body, and thus an accurate measurement of the pulse wave velocity can be performed without being influenced by the hydrostatic pressures caused according to the position when the measurement is performed in the sitting position, the upright position or the like that is not supine.
In a pulse wave velocity measurement device according to one embodiment, the pulse wave velocity correction unit comprises:

a reflection point height calculation unit for calculating a vertical height, of a reflection point where the pulse wave from a heart of the living body is reflected, with respect to the heart, and
a pulse wave velocity increment calculation unit for calculating an increment in the pulse wave velocity on basis of the vertical height of the reflection point that is calculated by the reflection point height calculation unit.

According to the embodiment, the reflection point height calculation unit calculates the vertical height, of the reflection point where the pulse wave from the heart is reflected, with respect to the heart of the living body and the pulse wave velocity increment calculation unit calculates the increment in the pulse wave velocity on basis of the calculated vertical height of the reflection point, and thus the pulse wave velocity measured in the sitting position or the upright position can be calculated so as to have a value equivalent to the pulse wave velocity measured in the supine position, even though the internal pressure and the pulse wave velocity are increased because the heart of the living body is positioned so as not to be level with the reflection point.
The reflection point height calculation unit sets the reflection point in vicinity of the iliac arteries or the abdominal aorta in the living body, for instance.
In a pulse wave velocity measurement device according to one embodiment, the pulse wave velocity increment calculation unit finds a hydrostatic pressure difference between the heart and the reflection point on basis of the vertical height of the reflection point that is calculated by the reflection point height calculation unit, and calculates the increment in the pulse wave velocity on basis of the hydrostatic pressure difference.
According to the embodiment, the pulse wave velocity increment calculation unit finds the hydrostatic pressure difference between the heart and the reflection point on basis of the vertical height of the reflection point that is calculated by the reflection point height calculation unit, and calculates the increment in the pulse wave velocity on basis of the hydrostatic pressure difference, so that accurate correction for the influence of the hydrostatic pressures on the pulse wave velocity can be attained.
In a pulse wave velocity measurement device according to one embodiment, the pulse wave velocity increment calculation unit calculates the increment in the pulse wave velocity on basis of a relational expression between a pulse wave velocity measured in advance when the living body is in a supine position and a pulse wave velocity measured in advance when the living body is in a sitting position or an upright position.

According to the embodiment, the pulse wave velocity increment calculation unit calculates the increment in the pulse wave velocity on basis of the relational expression between the pulse wave velocity measured in advance when the living body is in the supine position and the pulse wave velocity measured in advance when the living body is in the sitting position or the upright position, and thus the increment in the pulse wave velocity can easily be calculated in the subsequent measurement by the relational expression with use of the pulse wave velocity measured in advance in the supine position.

In a pulse wave velocity measurement device according to one embodiment, the pulse wave velocity correction unit comprises:

a measurement position detection unit for detecting a position of the living body on occasion of measurement, a reflection point height calculation unit for a calculating vertical height, of a reflection point where the pulse wave is reflected, with respect to a heart of the living body, on basis of the position of the living body on occasion of the measurement that is detected by the measurement position detection unit, and a pulse wave velocity increment calculation unit for calculating the increment in the pulse wave velocity on basis of the vertical height of the reflection point that is calculated by the reflection point height calculation unit.

According to the embodiment, the measurement position detection unit detects the position of the living body on occasion of the measurement, and the reflection point height calculation unit calculates the vertical height, of the reflection point where the pulse wave is reflected, with respect to the heart of the living body on basis of the detected position of the living body on occasion of the measurement. Thus the pulse wave velocity increment calculation unit calculates the increment in the pulse wave velocity on basis of the vertical height of the reflection point that is calculated by the reflection point height calculation unit, and accurate correction for the pulse wave velocity according to the measurement position of the living body can consequently be attained.

In a pulse wave velocity measurement device according to one embodiment, the measurement position detection unit detects a slope angle of a midline of the living body with respect to a horizontal plane, and wherein the reflection point height calculation unit calculates the vertical height of the reflection point that is calculated by the reflection point height calculation unit, on basis of the slope angle of the midline of the living body with respect to the horizontal plane that is detected by the measurement position detection unit.

According to the embodiment, the vertical height of the reflection point can easily be calculated with use of an angle sensor or the like for detecting the slope angle because the measurement position detection unit detects the slope angle of the midline of the living body with respect to the horizontal plane and because the reflection point height calculation unit calculates the vertical height, of the reflection point on basis of the detected slope angle of the midline of the living body with respect to the horizontal plane.

In a pulse wave velocity measurement device according to one embodiment, the pulse wave detection unit detects the pulse wave at one site in the living body, and wherein the pulse wave velocity calculation unit comprises:

a reference time detection unit for detecting a reference time for identification of an ejection wave component included in the pulse wave at the one site that is detected by the pulse wave detection unit and a reference time for identification of a reflected wave component included in the pulse wave, and a pulse wave amplitude detection unit for detecting an amplitude of the pulse wave that corresponds to the reference time for the ejection wave component detected by the reference time detection unit and detecting an amplitude of the pulse wave that corresponds to the reference time for the reflected wave component detected by the reference time detection unit, and the pulse wave velocity calculation unit calculates the velocity of the pulse wave on basis of the reference time for the ejection wave component and the reference time for the reflected wave component that are detected by the reference time detection unit, and the amplitude of the pulse wave corresponding to the reference time for the ejection wave component and the amplitude of the pulse wave corresponding to the reference time for the reflected wave component that are detected by the pulse wave amplitude detection unit.

According to the embodiment, the pulse wave velocity calculation unit calculates the velocity of the pulse wave on basis of the reference time for the ejection wave component and the reference time for the reflected wave component that are detected by the reference time detection unit and the amplitude of the pulse wave corresponding to the reference time for the ejection wave component and the amplitude of the pulse wave corresponding to the reference time for the reflected wave component which amplitudes are detected by the pulse wave amplitude detection unit, and the pulse wave velocity can consequently be measured with high accuracy in consideration of the difference in the pulse wave velocity between the ejection wave and the reflected wave due to the difference between the amplitude of the ejection wave component and the amplitude of the reflected wave component.

In a pulse wave velocity measurement method according to the present invention, the method comprises steps of:

detecting a pulse wave of a living body by a pulse wave detection unit,
calculating a pulse wave velocity by a pulse wave velocity calculation unit on basis of the pulse wave detected by the pulse wave detection unit, and
correcting, by a pulse wave velocity correction unit, the pulse wave velocity calculated by the pulse wave velocity calculation unit so as to eliminate an increment in the pulse wave velocity that results from hydrostatic pressures caused according to a position of the living body.

According to the above configuration, the pulse wave velocity calculated by the pulse wave velocity calculation unit is corrected by the pulse wave velocity correction unit. It is possible to eliminate the increment in the pulse wave velocity that results from the influence of the hydrostatic pressures caused according to the position of the living body is eliminated, and thus an accurate measurement of the pulse wave velocity can be performed without being influenced by the hydrostatic pressures caused according to the position of the living body when the measurement is performed in the sitting position, the upright position or the like that is not supine.

In a pulse wave velocity measurement program according to the present invention, the program causes a computer to execute
a pulse wave velocity calculating function of calculating a pulse wave velocity on basis of a pulse wave of a living body, and
a pulse wave velocity correcting function of correcting the pulse wave velocity calculated by the pulse wave velocity calculating function so as to eliminate an increment in the pulse wave velocity that results from hydrostatic pressures caused according to a position of the living body.

According to the above configuration, the pulse wave velocity calculating function and the pulse wave velocity correcting function are executed by the computer, the pulse wave velocity calculated by the pulse wave velocity calculating function is corrected by the pulse wave velocity correcting function so that the increment in the pulse wave velocity that results from the influence of the hydrostatic pressures caused according to the position of the living body is eliminated, and thus an accurate measurement of the pulse wave velocity can be performed without being influenced by the hydrostatic pressures caused according to the position of the living body when the measurement is performed in the sitting position, the upright position or the like that is not supine.

Advantageous Effects of Invention

According to the present invention, as is apparent from the above, the pulse wave velocity measurement device can be provided that are capable of accurate measurement of the pulse wave velocity without being influenced by the hydrostatic pressures caused according to the position when the measurement is performed in the sitting position, the upright position or the like that is not supine. Therefore, conventional necessity to lie in a supine position for measurement of the pulse wave velocity is eliminated, and the measurement is performed in the sitting position, the upright position or the like that is not supine, and thus convenience for a user can be improved.

BRIEF DESCRIPTION OF DRAWINGS

Fig. 1 is a block diagram for a pulse wave velocity measurement device in accordance with a first embodiment of the invention;
Fig. 2A is a waveform diagram showing a waveform of a pulse wave detected by a pulse wave detection unit of the pulse wave velocity measurement device;
Fig. 2B is a waveform diagram showing an accelerogram of the pulse wave detected by the pulse wave detection unit of the pulse wave velocity measurement device;
Fig. 3A is a waveform diagram showing third derivative waveform of the pulse wave detected by the pulse wave detection unit of the pulse wave velocity measurement device;
Fig. 3B is a waveform diagram showing fourth derivative waveform of the pulse wave detected by the pulse wave detection unit of the pulse wave velocity measurement device;
Fig. 4 is a diagram showing amplitude values of an ejection wave component and a reflected wave component in a conventional pulse wave velocity measurement device;

Fig. 5 is a waveform diagram showing a waveform of the pulse wave and the ejection wave component and the reflected wave component of the pulse wave;

Fig. 6 is a schematic representation schematically showing a path through which the ejection wave component of the pulse wave propagates in a human body and a path through which the reflected wave component thereof propagates in the human body;

Fig. 7 is a representation illustrating correction for influence of hydrostatic pressures on the pulse wave velocity that is effected by division of a path from a heart to a position of an abdominal aorta reflection point into parts of a minute height;

Fig. 8 is a block diagram for a pulse wave velocity measurement device in accordance with a second embodiment of the invention; and

Fig. 9 is a representation illustrating an angle between a median line of a subject and a horizontal plane.

DESCRIPTION OF EMBODIMENTS

Hereinbelow, a pulse wave velocity measurement device, a pulse wave velocity measurement method, and a pulse wave velocity measurement program of the invention will be described in detail with reference to embodiments shown in the drawings. A human body is treated as a living body in the embodiments, whereas other animals of which pulse waves of a circulatory system can be measured may be treated without limitation to human body.

[First Embodiment]

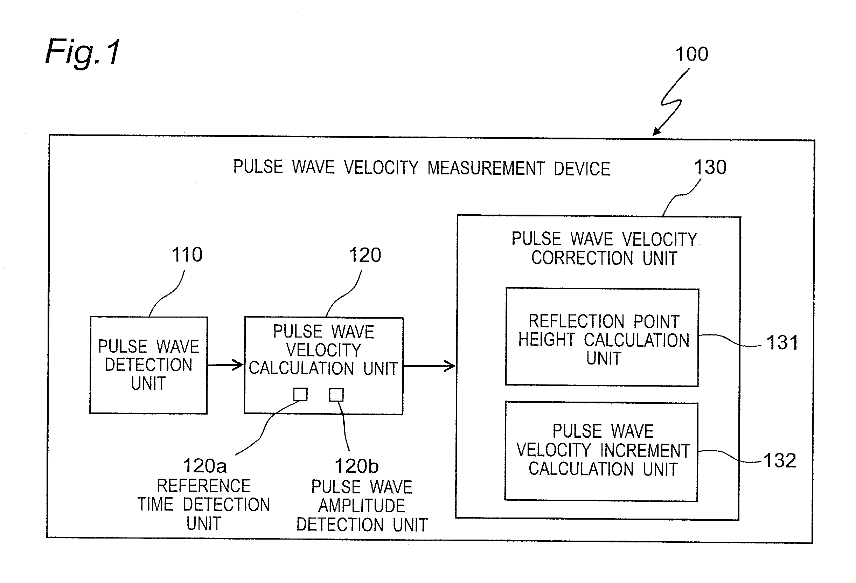

Fig. 1 shows a block diagram for a pulse wave velocity measurement device 100 in accordance with a first embodiment of the invention.

Main components of the pulse wave velocity measurement device 100 in accordance with the first embodiment are a pulse wave detection unit 110, a pulse wave velocity calculation unit 120, and a pulse wave velocity correction unit 130.

The pulse wave detection unit 110 detects pulse wave at one site in a human body. There are various methods by which the pulse wave detection unit 110 detects the pulse wave. For instance, there are photoplethysmography in which a degree of reflection or absorption of infrared red light outputted from a light emitting device according to a quantity of blood in a blood vessel is measured by a light receiving device, a pressure pulse wave method in which a change in pressure of blood in a blood vessel against the blood vessel is extracted as electric signals, and the like.

Especially severe limitations do not exist on the site in a living body where pulse waves are measured, whereas the site is desirably noninvasive and unconstrained site, if possible, and is preferably a finger tip, a wrist, an earlobe or the like of a human, for instance.

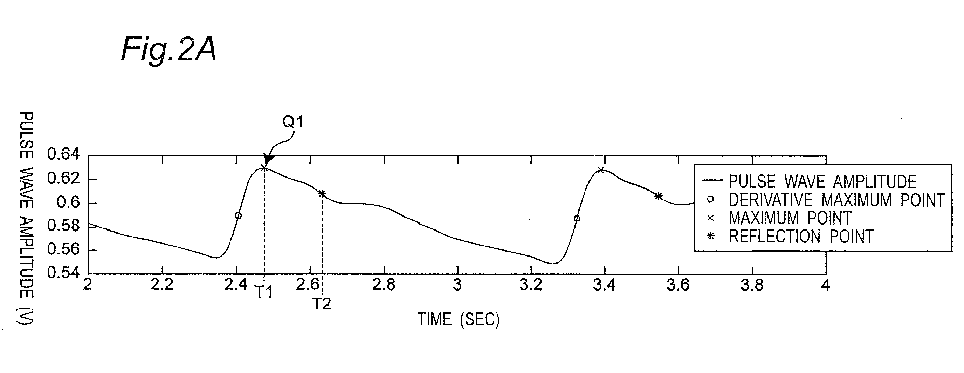

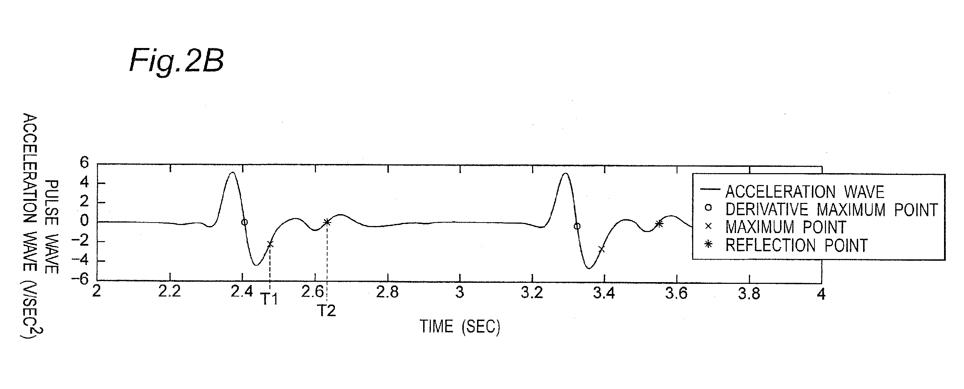

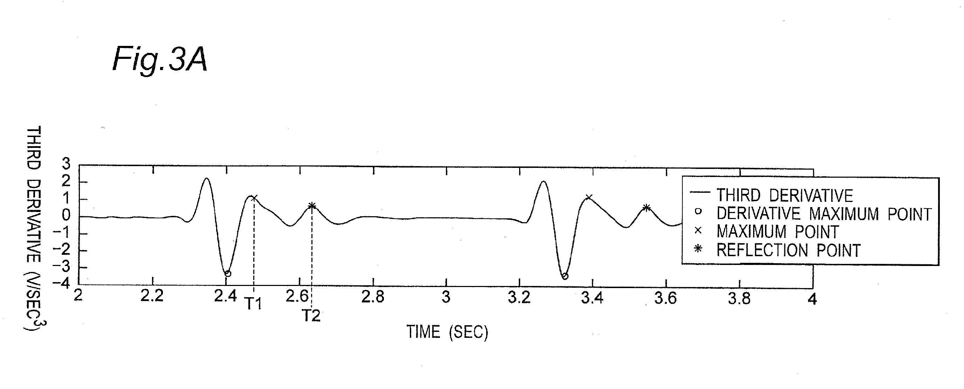

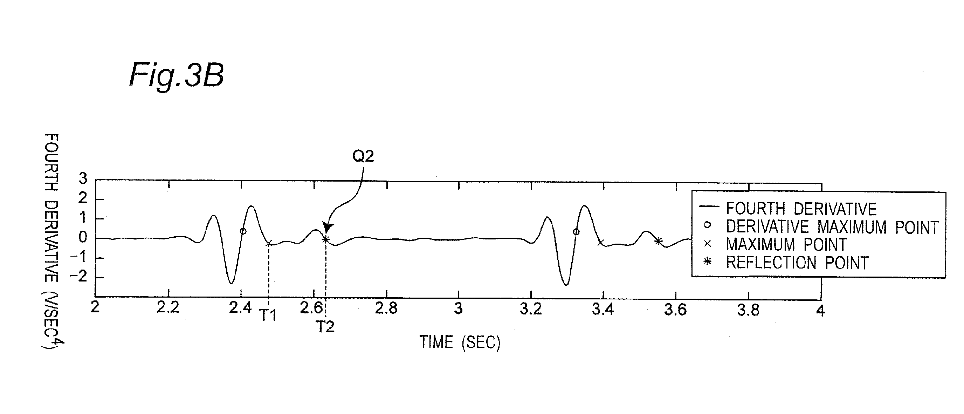

The pulse wave velocity calculation unit 120 has a reference time detection unit 120a and a pulse wave amplitude detection unit 120b. The reference time detection unit 120a finds respective reference times for identification of an ejection wave component and a reflected wave component that are included in pulse wave detected by the pulse wave detection unit 110. For Type C in a blood pressure waveform classification by Murgo et al., for instance, a time at a maximum point in a systole in a waveform of the pulse wave is detected as the reference time for the ejection wave component, and a third zero cross of a fourth derivative of the pulse wave is set as a reference point for the reflected wave component (see Figs. 2A, 2B, 3A, and 3B).

Pulse waves, however, exhibit a waveform other than the Type C of measured pulse waves or various waveforms according to noise level, age, sex, presence or absence of illness, physical conditions, and the like. In view of that, other suitable methods may be employed, if any, by which the time reference point for the ejection wave and the time reference point for the reflected wave can more preferably be found.

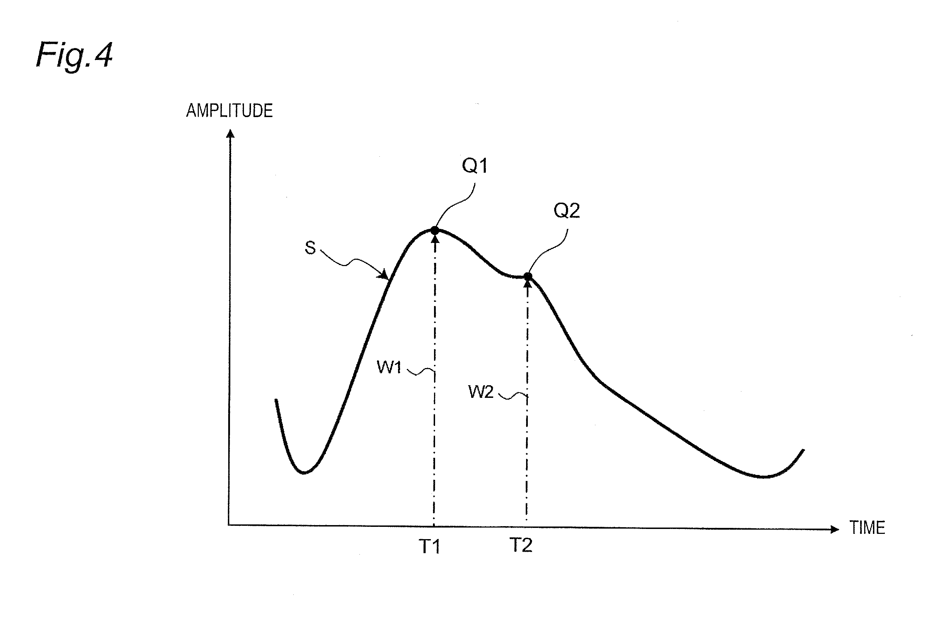

A pulse wave amplitude of the ejection wave component and a pulse wave amplitude of the reflected wave component are found by the pulse wave amplitude detection unit 120b. As shown in Fig. 4, for instance, an amplitude W1 that corresponds to the reference time T1 for the ejection wave component included in the pulse wave S detected by the reference time detection unit 120a is set as the pulse wave amplitude of the ejection wave component, and an amplitude W2 that corresponds to the reference time T2 for the reflected wave component is set as the pulse wave amplitude of the reflected wave component. These are methods employed for AI (Augmentation Index) that has recently been used as a diagnostic index for circulatory system.

Hereinbelow will be described an example of calculation of the pulse wave velocity that makes use of time information and pulse wave amplitude information of the ejection wave and the reflected wave.

Fig. 2A shows an example of a waveform of a pulse wave detected by the pulse wave detection unit 110. A vertical axis in Fig. 2A represents measured voltage values (V) corresponding to amplitudes (mmHg) of the pulse wave. In the pulse wave velocity measurement device 100 of the embodiment, correction (calibration) of correspondence of the voltage values (V) measured by the pulse wave detection unit 110 with the amplitudes (mmHg) of the pulse wave is performed on basis of a blood pressure (mmHg) measured by a conventional cuff-type sphygmomanometer, as an example. The correction (calibration) has only to be performed when first use is started, and result of the calibration has only to be used for the subsequent measurement.

On condition that the pulse wave is of Type C in the blood pressure waveform classification by Murgo et al., for instance, the reference time detection unit 120a detects time T1, at a maximum point Q1 in a systole in a waveform of the pulse wave shown in Fig. 2A, as reference time T1 for the ejection wave component. Fig. 2B shows acceleration wave of the pulse wave, and Fig. 3A shows a third derivative of the pulse wave. On condition that the pulse wave is of Type C in the blood pressure waveform classification by Murgo et al., for instance, the reference time detection unit 120a detects a third zero cross point Q2 of a fourth derivative of the pulse wave shown in Fig. 3B, as reference time T2 for the reflected wave component. The third zero cross point Q2 signifies a point where the fourth derivative waveform shown in Fig. 3B crosses zero downward at the third time after the pulse wave shown in Fig. 2A pass the minimum value.

Though the above description has been given on the detected pulse wave that is of Type C in the blood pressure waveform classification, a method by which the reference time for the ejection wave component of the pulse wave and the reference time for the reflected wave component thereof can more preferably be determined may be employed, if any, on condition that the detected pulse wave has a waveform other than Type C in the blood pressure waveform classification. For instance, basically, a pulse wave does not exhibit so great change in waveform unless there is great fluctuation in the blood pressure, and thus accuracy in detecting a pulse wave can be improved by superimposition (arithmetic mean) of a plurality of measured pulse waves.

Pulse waves exhibit various waveforms according to noise level, age, sex, presence or absence of illness, physical conditions, and the like, and thus a method by which the reference time for the ejection wave component of the pulse waves and the reference time for the reflected wave component thereof can more preferably be determined may be employed, if any. For instance, accuracy in determining the reference time for the ejection wave component of the pulse waves and the reference time for the reflected wave component thereof can be improved by recording, as a history, of the detected pulse waves along with conditions of a measured person at that point of time.

As shown in a waveform diagram of Fig. 4 as an example, the pulse wave amplitude detection unit 120b detects the amplitude W1 of the pulse wave S that corresponds to the reference time T1 for the ejection wave component detected by the reference time detection unit 120a and detects the amplitude W2 of the pulse wave S that corresponds to the reference time T2 for the reflected wave component detected by the reference time detection unit 120a.

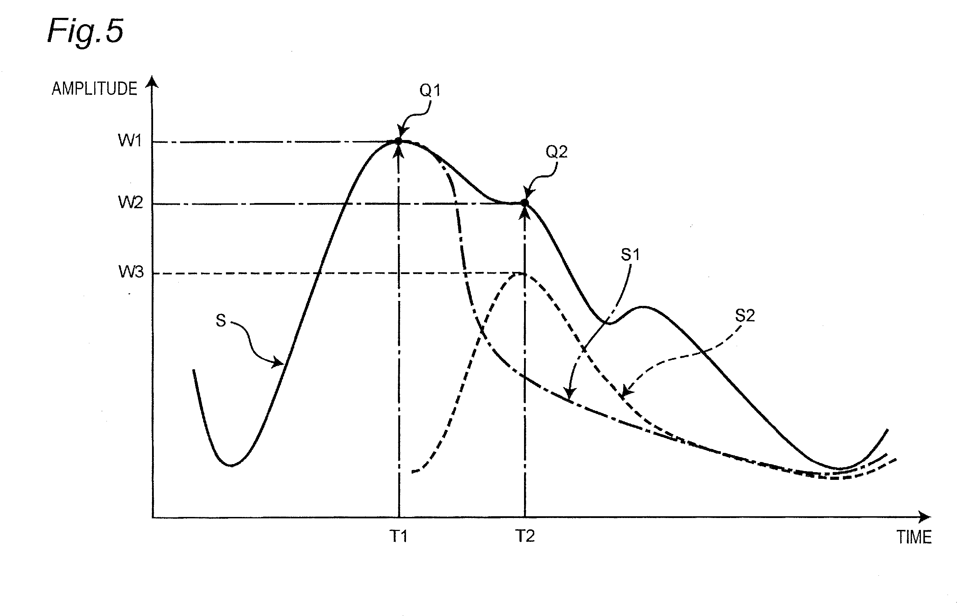

As shown in Fig. 5, the pulse wave velocity calculation unit 120 eliminates an ejection wave component S1 included in the amplitude W2 of the pulse wave S from the amplitude W2 of the pulse wave S, which amplitude corresponds to the reference time T2 for a reflected wave component S2 and thereby finds an amplitude W3 of the reflected wave component S2, that corresponds to the reference time T2 for the reflected wave component S2. As a method of finding the amplitude W3 of the reflected wave component S2 that corresponds to the reference time T2 for the reflected wave component S2, a method is conceivable in which degrees of decrease in the ejection waves in the pulse waves are modeled with use of Windkessel model, for instance, and in which a remaining ejection wave component S1 is subtracted from the pulse wave amplitude measured around the reference time T2 for the reflected wave component S2. As a matter of course, a method by which the remaining ejection wave component S1 can more accurately be determined may be employed, if any.

The pulse wave velocity calculation unit 120 finds the velocity of the pulse wave S on basis of the reference time T1 for the ejection wave component, the reference time T2 for the reflected wave component, the amplitude W1 of the pulse wave S that corresponds to the reference time T1 for the ejection wave component, and the amplitude W3 of the reflected wave component S2 of the pulse wave S that corresponds to the reference time T2 for the reflected wave component S2.

Subsequently, a process will be described in which the pulse wave velocity calculation unit 120 finds the velocity PWV of the pulse wave S.

For the pulse wave velocity, in general, pulse waves at two sites in a living body are measured, velocities at which the ejection wave components of the pulse waves are propagated are found by a fundamental principle that is based on the principle of Moens Korteweg. A relation between the pulse wave velocity and the blood pressure is derived from the following equation (1) on basis of the relation of Moens Korteweg (see McCombie, Devin "Development of a wearable blood pressure monitor using adaptive calibration of peripheral pulse transit time measurements", Ph.D. Thesis, Massachusetts Institute of Technology, Dept. of Mechanical Engineering, 2008).

$$(PWV)^2 = \alpha \cdot \exp(\beta \times P) \quad \ldots \quad (1)$$

In the equation (1), PWV is the pulse wave velocity (m/sec), P is the blood pressure (mmHg), and $\alpha$, $\beta$ are constants that slightly change among individuals and according to measurement time even in an individual.

By application of the equation (1) to pulse wave velocity PWV1 (m/sec) of the ejection waves S1 and to pulse wave velocity PWV2 (m/sec) of the reflected waves S2, the following equations (2) and (3) are obtained, respectively.

$$(PWV1)^2 = \alpha \cdot \exp(\beta \times W1) \quad \ldots \quad (2)$$

$$(PWV2)^2 = \alpha \cdot \exp(\beta \times W3) \quad \ldots \quad (3)$$

In the equation (2), W1 is the pulse wave amplitude of the ejection wave S1, that is, the amplitude W1 of the pulse wave S that corresponds to the reference time T1 for the ejection wave S1 in Fig, 5, and the amplitude W1 corresponds to a pulse wave pressure of the ejection wave S1. W3 is the pulse wave amplitude of the reflected wave S2, that is, the amplitude W3 of the reflected wave S2 that corresponds to the reference time T2 for the reflected wave S2 in Fig, 5, and the amplitude W3 corresponds to a pulse wave pressure of the reflected wave S2.

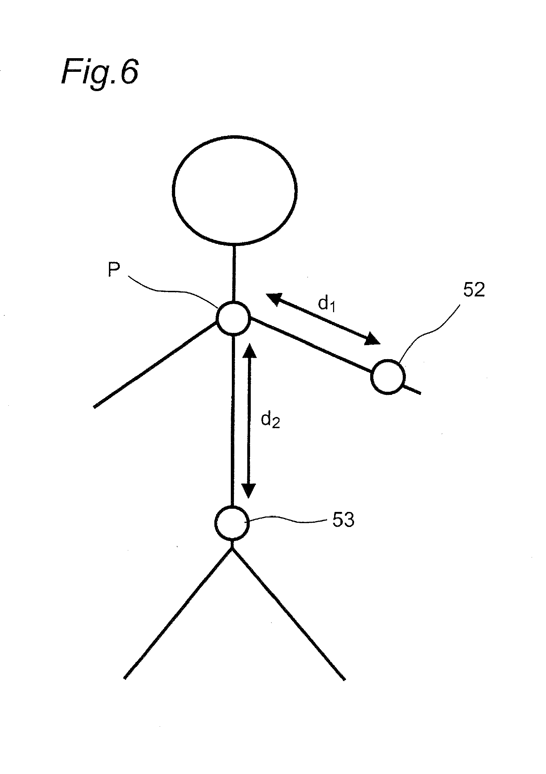

From designation of a distance from a heart P of a human body to a pulse wave measurement point 52 as d1 (m), a distance from the heart P to a reflection point 53 as d2(m), a time between a pulsation of the heart P and the reference time T1 for the ejection wave S1 as $\Delta T1$ (sec), and a time difference (T2-T1) between the reference time T1 for the ejection wave S1 and the reference time T2 for the reflected wave S2 as $\Delta T2$ (sec), as shown in Fig. 6, the following equations (4) and (5) are obtained.

$$(PWV1)^2 = (d_1/\Delta T1)^2 \qquad \ldots \quad (4)$$

$$(PWV2)^2 = ((2d_2+d_1)/(\Delta T1+\Delta T2))^2 \quad \ldots \quad (5)$$

The following equation (6) is obtained from the equations (2) and (4), and the following equation (7) is obtained from the equations (3) and (5).

$$\alpha \cdot \exp(\beta \times W1) = (d_1/\Delta T1)^2 \qquad \ldots \quad (6)$$

$$\alpha \cdot \exp(\beta \times W3) = ((2d_2+d_1)/(\Delta T1+\Delta T2))^2 \quad \ldots \quad (7)$$

The time $\Delta T1$ can be eliminated from the equations (6) and (7). That is, the following equation (8) is obtained from the equation (7).

$$(\Delta T1+\Delta T2)^2 = (2d_2+d_1)^2/\alpha \cdot \exp(\beta \times W3)$$

$$\Delta T1+\Delta T2 = (2d_2+d_1)/\{\alpha \cdot \exp(\beta \times W3)\}^{1/2}$$

$$\Delta T1 = (2d_2+d_1)/\{\alpha \cdot \exp(\beta \times W3)\}^{1/2}-\Delta T2 \quad \ldots \quad (8)$$

Substitution of the equation (8) for the equation (4) provides the following equation (9).

$$(PWV1)^2 = d^2 \times [(2d_2+d_1)/\{\alpha \cdot \exp(\beta \times W3)\}^{1/2} - \Delta T2]^{-2}$$

$$PWV1 = d \times [(2d_2+d_1)/\alpha \cdot \exp(\beta \times W3)]^{1/2} - \Delta T2]^{-1} \quad \ldots (9)$$

That is, the pulse wave velocity PWV1 of the ejection wave S1 can be calculated from the known constants $\alpha$, $\beta$, the distances $d_1$, $d_2$ that are known measurements, the difference $\Delta T2$ (= T2-T1) between the reference times T2 and T1 found by the reference time detection unit 120a, and the amplitude W3 of the reflected wave S2, found by the ejection wave component elimination unit 4, that corresponds to the reference time T2 for the reflected wave S2.

That is, the pulse wave velocity calculation unit 120 finds the velocity PWV1 of the ejection wave S1 of the pulse wave S, with use of the equation (9) derived on basis of the equations (4) through (7) as described above, on basis of the difference $\Delta T2$ between the reference times T1 and T2 for the ejection wave component S1 and for the reflected wave component S2, and the amplitudes W1, W3 of the ejection wave component S1 and the reflected wave component S2 of the pulse wave S that correspond to the reference time T2 for the reflected wave component S2. Accordingly, the pulse wave velocity can be measured with high accuracy in consideration of a difference in the pulse wave velocity between the ejection wave and the reflected wave due to a difference between the amplitude W1 of the ejection wave component S1 and the amplitude W3 of the reflected wave component S2.

The embodiment in which the pulse wave amplitude detection unit 120b uses the amplitude W3 of the ejection waves S2 has been described above, whereas the pulse wave amplitude detection unit 120b that does not use the amplitude W3 of the ejection wave S2 will use the following equation (10) that employs the amplitude W2 of the pulse wave S corresponding to the reference time T2 for the reflected wave S2 instead of the amplitude W3 of the reflected wave S2 corresponding to the reference time T2 for the reflected wave S2 in the equation (3).

$$(PWV2)^2 = \alpha \cdot \exp(\beta \times W2) \quad \ldots (10)$$

In this example, the following equation (11) is obtained from the equations (10) and (5).

$$\alpha \cdot \exp(\beta \times W2) = ((2d_2+d_1)/(\Delta T1+\Delta T2))^2 \quad \ldots (11)$$

Accordingly, elimination of the time $\Delta T1$ from the equation (6) described above and the equation (11) provides the following equation (12).

$$\Delta T1 = (2d_2+d_1)/\{\alpha \cdot \exp(\beta \times W2)\}^{1/2} - \Delta T2 \quad \ldots (12)$$

Substitution of the equation (12) into the above described equation (4) provides the following equation (13).

$$(PWV1)^2 = d_1^2 \times [(2d_2+d_1)/\{\alpha \cdot \exp(\beta \times W2)\}^{1/2} - \Delta T2]^{-2}$$

$$PWV1 = d_1 \times [(2d_2+d_1)/\alpha \cdot \exp(\beta \times W2)]^{1/2} - \Delta T2]^{-1} \quad \ldots (13)$$

That is, the pulse wave velocity PWV1 of the ejection wave S1 can be calculated from the known constants $\alpha$, $\beta$, the distances $d_1$, $d_2$ that are the known measurements, the difference $\Delta T2$ (= T2-T1) between the reference times T2 and T1 found by the reference time detection unit 120a, and the amplitude W2 of the pulse wave S that corresponds to the reference time T2 for the reflected wave S2 found by the pulse wave amplitude detection unit 120b.

That is, the pulse wave velocity calculation unit 120 finds the velocity PWV1 of the ejection wave S1 of the pulse wave S, with use of the equation (13) derived as described above, on basis of the difference $\Delta T2$ between the reference times T1 and T2 for the ejection wave component S1 and for the reflected wave component S2, and the amplitude W2 of the pulse wave S at the reference time T2 that corresponds to the reference time T2 for the reflected wave component S2. Accordingly, the pulse wave velocity can be measured with high accuracy in consideration of the difference in the pulse wave velocity between the ejection wave S1 and the reflected wave S2 of the pulse wave S.

Though the device for measuring the pulse wave velocity has been described for the embodiment, a blood pressure can be measured on basis of the pulse wave velocity measured in the embodiment, as is apparent from the equation (1). That is, the pulse wave velocity obtained from the pulse wave velocity measurement device may be displayed as a blood pressure value instead of the pulse wave velocity displayed as it is, when a user uses the pulse wave velocity measurement device. That is because the blood pressure is a living-body index more familiar to non-medical personnel than the pulse wave velocity though the pulse wave velocity is a living-body index extremely familiar to medical personnel.

It takes several tens of seconds for a conventional sphygmomanometer using a cuff to display a set of a systolic blood pressure, a diastolic blood pressure, a mean blood pressure, a pulse rate and/or the like to a user, whereas a sphygmomanometer based on the embodiment of the invention is capable of detecting a blood pressure for each pulsation and there is a possibility that it is capable of grasping conditions of a living body in greater detail. The pulse wave velocity and the blood pressure can be detected with higher accuracy by acquisition of an arithmetic mean, a moving average and/or the like for several pulsations as a unit.

The pulse wave velocity correction unit 130 has a reflection point height calculation unit 131 and a pulse wave velocity increment calculation unit 132. The pulse wave velocity correction unit 130 corrects an influence of hydrostatic pressures that arises in the measurement of the pulse wave velocity in the sitting position or the upright position in comparison with that in the supine position.

In the conventional pulse wave velocity measurement devices, a measurement is normally performed in the supine position. Then the heart is level with the abdominal aorta that is the reflection point (point where a pulse wave from the heart is reflected), so that hydrostatic pressures exert no influence upon the pulse wave velocity. When the measurement is performed in the sitting position or the upright position, however, the heart is not level with the abdominal aorta that is the reflection point and an internal pressure increases all the more because the influence of the hydrostatic pressures is exerted thereon. The increase in the internal pressure in a blood vessel causes increase in hardness of the blood vessel, which increase causes increase in the pulse wave velocity.

In the first embodiment, accordingly, the correction for the pulse wave velocities is made by the pulse wave velocity correction unit 130 so that the correction may be made for a velocity difference between the pulse wave velocity measured in the sitting position (or the upright position) and the pulse wave velocity measured in the supine position and so that the pulse wave velocity in the sitting position (or the upright position) can be calculated so as to have a value equivalent to the pulse wave velocity measured in the supine position.

The reflection point height calculation unit 131 calculates the position of the reflection point in vicinity of the iliac arteries or the abdominal aorta in a human body. For the calculation of the position of the reflection point, a relation between "body height or sitting height" and "vertical height from the heart to the position of the reflection point in vicinity of the iliac arteries or the abdominal aorta" is defined in advance by a table, a relational expression or the like in the reflection point height calculation unit 131. On basis of information of a body height or a sitting height of a measured person that is inputted into the pulse wave velocity measurement device 100, the reflection point height calculation unit 131 calculates the vertical height of the reflection point with respect to the heart by using the table, the relational expression or the like that have been predefined.

The pulse wave velocity increment calculation unit 132 calculates an increment in the pulse wave velocity that results from the influence of the hydrostatic pressures, on basis of the vertical height of the reflection point that is found by the reflection point height calculation unit 131.

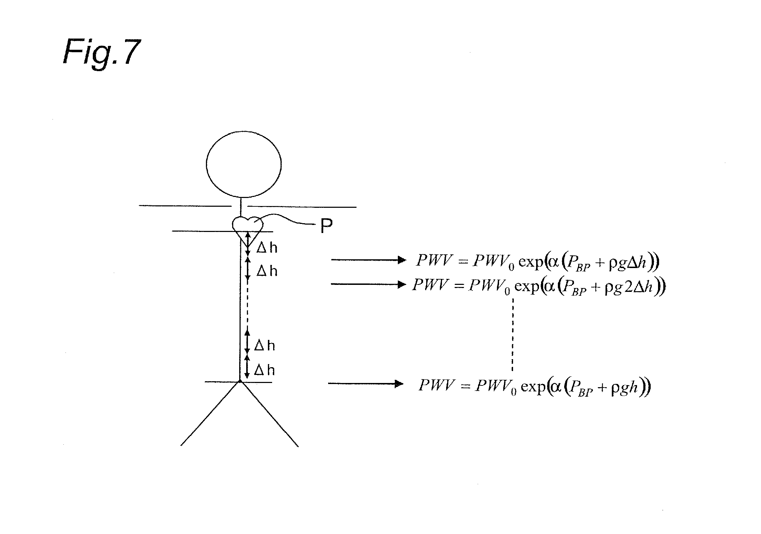

In a method of correction for the influence of the hydrostatic pressures, as shown in Fig. 7, the correction for the influence of the hydrostatic pressures on the pulse wave velocity is carried out by a division of the vertical height from the heart P to the position of the reflection point by a minute height $\Delta h$ and then an integration of the influences of the hydrostatic pressures at the resultant positions. Thus the pulse wave velocity in a range in the path from the heart to the position of the reflection point can be expressed by the following equation (14) on basis of a blood pressure value measured at a height of the heart and a vertical height from the heart to the range.

$$PWV = \beta \cdot \exp(\alpha(P_{BP} + \rho g h) \quad \dots \quad (14)$$

wherein

PWV is the pulse wave velocity [m/s],
$\alpha$, $\beta$ are constants (that slightly change among individuals and from time to time for the measurement in each individual),
$P_{BP}$ is the blood pressure value measured at the height of the heart,

p is density of blood,
g is acceleration of gravity, and
h is the vertical height from the heart to the range.

The increment in the pulse wave velocity that results from the influence of the hydrostatic pressures can be expressed by the following equation (15).

$$\Delta PWV = PWV_0 \cdot \exp(\alpha P_{BP}) \cdot (\exp(\alpha\rho gh)-1) \quad \ldots \quad (15)$$

wherein $PWV_0$ is a pulse wave velocity at the height of the heart.

Then $\Delta PWV$ is calculated for each of the divided ranges and a mean value of the calculated $\Delta PMV$ is obtained as a correction value.

In the pulse wave velocity increment calculation unit 132, subsequently, the pulse wave velocity that would be measured in the supine position can be calculated by subtraction of the mean value of $\Delta PWV$ from the pulse wave velocity calculated by the pulse wave velocity calculation unit 120. The smaller the minute height $\Delta h$ is, the more accurate value can be calculated; however, it is desirable to divide the vertical height from the heart to the position of the reflection point into ten ranges, for instance.

In the pulse wave velocity measurement device 100 having the above configuration, the pulse wave velocity calculated by the pulse wave velocity calculation unit 120 is corrected by the pulse wave velocity correction unit 130 so that the increment in the pulse wave velocity that results from the influence of the hydrostatic pressures caused according to the position of the human body is eliminated, and thus accurate measurement of the pulse wave velocity can be performed without being influenced by the hydrostatic pressures caused according to the position when the measurement is performed in the sitting position or the upright position that is not supine.

The reflection point height calculation unit 131 of the pulse wave velocity correction unit 130 calculates the vertical height of the reflection point with respect to the heart, the pulse wave velocity increment calculation unit 132 of the pulse wave velocity correction unit 130 calculates the increment in the pulse wave velocity on basis of the calculated vertical height of the reflection point, and thus the pulse wave velocity measured in the sitting position or the upright position can be calculated so as to have a value equivalent to the pulse wave velocity measured in the supine position, even though the pulse wave velocity is increased because the heart is positioned so as not to be level with the reflection point, and thus the internal pressure is increased.

Accurate correction for the influence of the hydrostatic pressures on the pulse wave velocity can be attained by the pulse wave velocity increment calculation unit 132 on basis of the vertical height of the reflection point that is calculated by the reflection point height calculation unit 131.

The pulse wave velocity calculation unit 120 calculates the velocity of pulse wave on basis of the reference time for the ejection wave component and the reference time for the reflected wave component that are detected by the reference time detection unit 120a of the pulse wave velocity calculation unit 120 and the amplitude of the pulse wave corresponding to the reference time for the ejection wave component and the amplitude of the pulse wave corresponding to the reference time for the reflected wave component that are detected by the pulse wave amplitude detection unit 120b, and thus the pulse wave velocity can be measured with high accuracy in consideration of the difference in the pulse wave velocity between the ejection wave and the reflected wave due to the difference between the amplitude of the ejection wave component and the amplitude of the reflected wave component.

The increment in the pulse wave velocity that results from the influence of the hydrostatic pressures is calculated with use of the equations (14) and (15) in the first embodiment, whereas there is no limitation thereto and the increment in the pulse wave velocity has only to be calculated on basis of the relational expression between the pulse wave velocity measured in advance when the human body is in the supine position and the pulse wave velocity measured when the human body is in the sitting position (or the upright position).

For instance, the pulse wave velocity increment calculation unit 132 measures, in advance, pulse wave velocities on two conditions, i.e., in the supine position and in the sitting position (or the upright position), and calculates a difference $\Delta PWV$ (= PWVa-PWVb) between the pulse wave velocity PWVa measured in the supine position and the pulse wave velocity PWVb measured in the sitting position (or the upright position). A value of the difference $\Delta PWV$ (= PWVa-PWVb) is stored in a storage unit (not shown) of the pulse wave velocity measurement device 100, and thus the difference $\Delta PWV$ stored in the storage unit is subtracted from the value of the pulse wave velocity calculated by the pulse wave velocity calculation unit 120, in the subsequent and later measurement.

Thus the pulse wave velocity increment calculation unit 132 calculates the increment in the pulse wave velocity on basis of the difference $\Delta PWV = PWVa-PWVb$ that is the relational expression between the pulse wave velocity measured in advance when the human body is in the supine position and the pulse wave velocity measured when the human body is in the sitting position (or the upright position), and the calculation can easily be attained in the subsequent measurement by the relational expression with use of the pulse wave velocities measured in advance in the supine position.

The relational expression between the pulse wave velocity in the supine position and the pulse wave velocity in the sitting position (or the upright position) is not limited thereto and has only to be a relational expression that represents a correlation between the pulse wave velocity in the supine position and the pulse wave velocity in the sitting position (or the upright position).

[Second Embodiment]

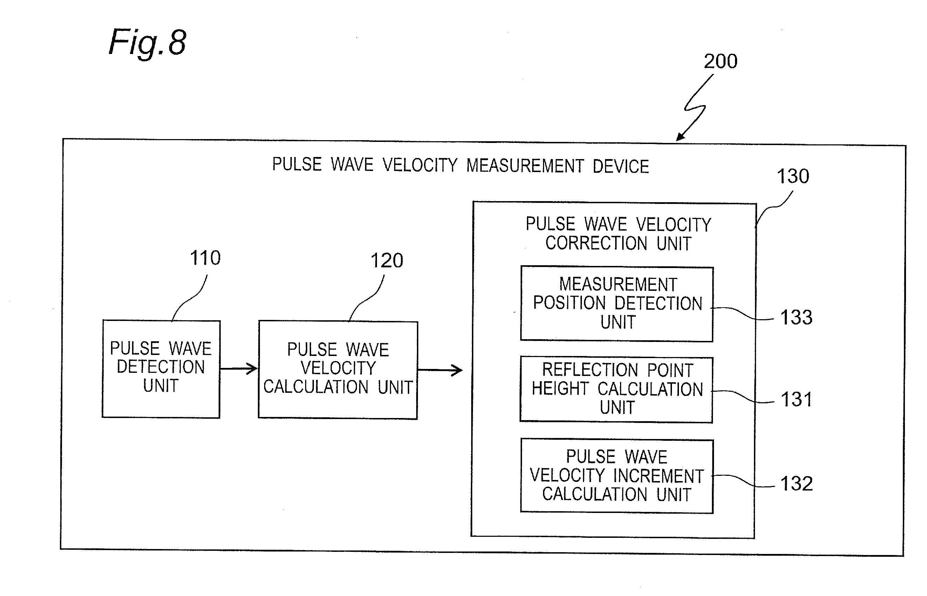

Fig. 8 shows a block diagram for a pulse wave velocity measurement device 200 in accordance with a second embodiment of the invention. The pulse wave velocity measurement device 200 in accordance with the second embodiment has the same configuration as the pulse wave velocity measurement device 100 in accordance with the first embodiment has, except for a measurement position detection unit 133.

The pulse wave velocity measurement device 200 in accordance with the second embodiment has the pulse wave detection unit 110, the pulse wave velocity calculation unit 120, and the pulse wave velocity correction unit 130.

The pulse wave velocity correction unit 130 is composed of the measurement position detection unit 133, the reflection point height calculation unit 131, and the pulse wave velocity increment calculation unit 132.

The measurement position detection unit 133 detects a measurement position of a measured person. A change in the measurement position involves a change in a vertical height of the reflection point with respect to the heart. Therefore, a measurement of the change in the vertical height of the reflection point makes it possible to calculate a value equivalent to a pulse wave velocity measured in the supine position without an influence on the measurement position of the measured person.

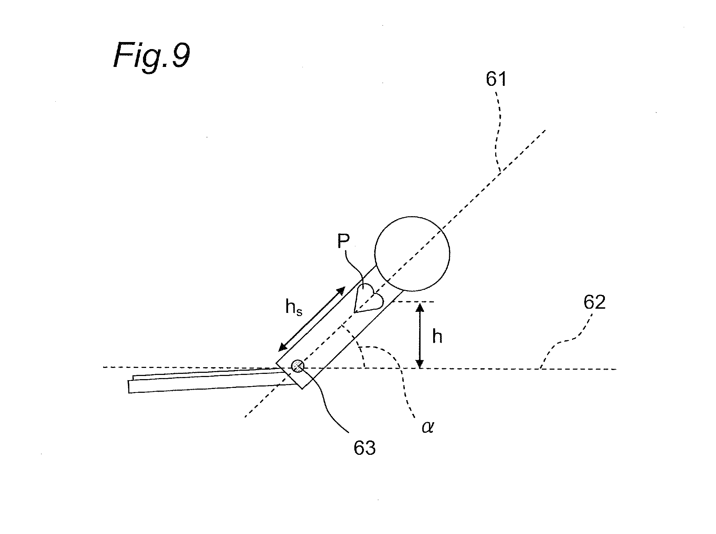

As shown in Fig. 9, the measurement position detection unit 133 calculates an angle $\alpha$ (slope angle) between a median line 61 of a subject and a horizontal plane 62, on basis of information on acceleration of gravity detected by an acceleration sensor attached onto a waist.

The reflection point height calculation unit 131 finds the vertical height h from the heart P to the reflection point 63 by using the following equation (16) on basis of the angle $\alpha$ obtained from the measurement position detection unit 133 and a distance hs from the heart P to the reflection point 63.

$$h = hs \cdot \sin\alpha \quad \ldots \quad (16)$$

The pulse wave velocity correction unit 130 corrects the pulse wave velocity on basis of the vertical height h found by the reflection point height calculation unit 131. A manner of the correction by the pulse wave velocity correction unit 130 is the same as that of the first embodiment.

The pulse wave velocity measurement device in accordance with the second embodiment has the same effects as the pulse wave velocity measurement device in accordance with the first embodiment has.

Accurate correction for the pulse wave velocity according to a measurement position of a human body can be attained by the measurement position detection unit 133 that detects the position of the human body on occasion of the measurement, by the reflection point height calculation unit 131 that calculates the vertical height, of the reflection point where the pulse waves are reflected, with respect to the heart of the human body on basis of the detected position of the human body on occasion of the measurement, and by the pulse wave velocity increment calculation unit 132 that calculates the increment in the pulse wave velocity on basis of the vertical height h of the reflection point calculated by the reflection point height calculation unit 131.

The height of the reflection point can easily be calculated with use of an angle sensor for detecting a slope angle because the measurement position detection unit 133 detects the slope angle of the midline of the human body with respect to the horizontal plane and because the reflection point height calculation unit 131 calculates the height of the reflection point on basis of the detected slope angle of the midline of the human body with respect to the horizontal plane.

Though the pulse wave velocity measurement devices including the pulse wave velocity calculation unit 120 for calculating the pulse wave velocity on basis of the reference times and amplitudes of the ejection wave component and the reflected wave component of the pulse wave at one site in the human body have been described for the first and second embodiments, the pulse wave velocity calculation unit is not limited thereto and may be a unit that calculates the pulse wave velocity by another method. For instance, the invention may be applied to pulse wave velocity measurement devices in which pulse waves at two points, i.e., a pulse wave at an ankle and a pulse wave on an upper arm are used and to pulse wave velocity measurement devices in which an electrocardiogram and a pulse wave at another site are used.

The invention is not limited to the pulse wave velocity measurement devices and may be provided as a pulse wave velocity measurement method including steps of detecting a pulse wave of a living body by the pulse wave detection unit, calculating the pulse wave velocity by the pulse wave velocity calculation unit on basis of the pulse wave detected by the pulse wave detection unit, and correcting by the pulse wave velocity correction unit, the pulse wave velocity calculated by the pulse wave velocity calculation unit, so as to eliminate the increment in the pulse wave velocity that results from the hydrostatic pressures caused according to the position of the living body.

In the pulse wave velocity measurement method, the pulse wave velocity calculated by the pulse wave velocity calculation unit is corrected by the pulse wave velocity correction unit so that the increment in the pulse wave velocity that results from the influence of the hydrostatic pressures caused according to the position of the living body is eliminated, and thus accurate measurement of the pulse wave velocity can be performed without being influenced by the hydrostatic pressures caused according to the position of the living body when the measurement is performed in the sitting position, the upright position or the like that is not supine.

In the invention, a pulse wave velocity calculating function of calculating a pulse wave velocity on basis of a pulse wave of a living body and a pulse wave velocity correcting function of correcting the pulse wave velocity calculated by the pulse wave velocity calculating function so as to eliminate an increment in the pulse wave velocity that results from the hydrostatic pressures caused according to a position of the living body may be executed by a computer with use of a pulse wave velocity measurement program.

By the pulse wave velocity measurement program, the pulse wave velocity calculating function and the pulse wave velocity correcting function are executed by the computer, the pulse wave velocity calculated by the pulse wave velocity calculating function is corrected by the pulse wave velocity correcting function so that the increment in the pulse wave velocity that results from the influence of the hydrostatic pressures caused according to the position of the living body is eliminated, and thus accurate measurement of the pulse wave velocity can be performed without being influenced by the hydrostatic pressures caused according to the position when the measurement is performed in the sitting position, the upright position or the like that is not supine.

Though the specific embodiments of the invention have been described, the invention is not limited to the first and second embodiments and may be embodied with various modifications within the scope of the invention.

REFERENCE SIGNS LIST

100, 200    pulse wave velocity measurement device

110    pulse wave detection unit

120    pulse wave velocity calculation unit

120a    reference time detection unit

120b    pulse wave amplitude detection unit

130    pulse wave velocity correction unit

131    reflection point height calculation unit

132    pulse wave velocity increment calculation unit

133    measurement position detection unit

**Claims**

**1.** A pulse wave velocity measurement device comprising:

a pulse wave detection unit (110) for detecting a pulse wave in a living body,
a pulse wave velocity calculation unit (120) for calculating a pulse wave velocity on basis of the pulse wave detected by the pulse wave detection unit (110), and

a pulse wave velocity correction unit (130) for correcting the pulse wave velocity calculated by the pulse wave velocity calculation unit (120) so as to eliminate an increment in the pulse wave velocity that results from influence of hydrostatic pressures caused according to a position of the living body.

2. The pulse wave velocity measurement device as claimed in Claim 1, wherein
the pulse wave velocity correction unit (130) comprises:

a reflection point height calculation unit (131) for calculating a vertical height, of a reflection point where the pulse wave from a heart of the living body is reflected, with respect to the heart, and
a pulse wave velocity increment calculation unit (132) for calculating an increment in the pulse wave velocity on basis of the vertical height of the reflection point that is calculated by the reflection point height calculation unit (131).

3. The pulse wave velocity measurement device as claimed in Claim 2, wherein
the pulse wave velocity increment calculation unit (132) finds a hydrostatic pressure difference between the heart and the reflection point on basis of the vertical height of the reflection point that is calculated by the reflection point height calculation unit (131), and calculates the increment in the pulse wave velocity on basis of the hydrostatic pressure difference.

4. The pulse wave velocity measurement device as claimed in Claim 2, wherein
the pulse wave velocity increment calculation unit (132) calculates the increment in the pulse wave velocity on basis of a relational expression between a pulse wave velocity measured in advance when the living body is in a supine position and a pulse wave velocity measured in advance when the living body is in a sitting position or an upright position.

5. The pulse wave velocity measurement device as claimed in Claim 1, wherein
the pulse wave velocity correction unit (130) comprises:

a measurement position detection unit (133) for detecting a position of the living body on occasion of measurement,
a reflection point height calculation unit (131) for calculating a vertical height, of a reflection point where the pulse wave is reflected, with respect to a heart of the living body, on basis of the position of the living body on occasion of the measurement that is detected by the measurement position detection unit (133), and
a pulse wave velocity increment calculation unit (132) for calculating the increment in the pulse wave velocity on basis of the vertical height of the reflection point that is calculated by the reflection point height calculation unit (131).

6. The pulse wave velocity measurement device as claimed in Claim 5, wherein
the measurement position detection unit (133) detects a slope angle of a midline of the living body with respect to a horizontal plane, and wherein
the reflection point height calculation unit (131) calculates the vertical height of the reflection point that is calculated by the reflection point height calculation unit (131), on basis of the slope angle of the midline of the living body with respect to the horizontal plane that is detected by the measurement position detection unit (133).

7. The pulse wave velocity measurement device as claimed in any one of Claims 1 through 6, wherein
the pulse wave detection unit (110) detects the pulse wave at one site in the living body, and wherein
the pulse wave velocity calculation unit (120) comprises:

a reference time detection unit (120a) for detecting a reference time for identification of an ejection wave component included in the pulse wave at the one site that is detected by the pulse wave detection unit (110) and a reference time for identification of a reflected wave component included in the pulse wave, and
a pulse wave amplitude detection unit (120b) for detecting an amplitude of the pulse wave that corresponds to the reference time for the ejection wave component detected by the reference time detection unit (120a) and detecting an amplitude of the pulse wave that corresponds to the reference time for the reflected wave component detected by the reference time detection unit (120a), and
the pulse wave velocity calculation unit (120) calculates the velocity of the pulse wave on basis of the reference time for the ejection wave component and the reference time for the reflected wave component that are detected by the reference time detection unit (120a), and the amplitude of the pulse wave corresponding to the reference time for the ejection wave component and the amplitude of the pulse wave corresponding to the reference time for the reflected wave component that are detected by the pulse wave amplitude detection unit (120b).

8. A pulse wave velocity measurement method comprising steps of:

detecting a pulse wave of a living body by a pulse wave detection unit,
calculating a pulse wave velocity by a pulse wave velocity calculation unit on basis of the pulse wave detected by the pulse wave detection unit, and
correcting, by a pulse wave velocity correction unit, the pulse wave velocity calculated by the pulse wave velocity calculation unit so as to eliminate an increment in the pulse wave velocity that results from hydrostatic pressures caused according to a position of the living body.

9. A pulse wave velocity measurement program that causes a computer to execute
a pulse wave velocity calculating function of calculating a pulse wave velocity on basis of a pulse wave of a living body, and
a pulse wave velocity correcting function of correcting the pulse wave velocity calculated by the pulse wave velocity calculating function so as to eliminate an increment in the pulse wave velocity that results from hydrostatic pressures caused according to a position of the living body.

# Fig.1

100

PULSE WAVE VELOCITY MEASUREMENT DEVICE

130

PULSE WAVE VELOCITY CORRECTION UNIT

110

PULSE WAVE DETECTION UNIT

120

PULSE WAVE VELOCITY CALCULATION UNIT

REFLECTION POINT HEIGHT CALCULATION UNIT — 131

PULSE WAVE VELOCITY INCREMENT CALCULATION UNIT — 132

120a
REFERENCE TIME DETECTION UNIT

120b
PULSE WAVE AMPLITUDE DETECTION UNIT

*Fig.2A*

## Fig.2B

## Fig.3A

Fig.3B

*Fig.4*

*Fig.5*

## Fig.6

Fig.7

$$PWV = PWV_0 \exp(\alpha(P_{BP} + \rho g \Delta h))$$

$$PWV = PWV_0 \exp(\alpha(P_{BP} + \rho g 2\Delta h))$$

$$PWV = PWV_0 \exp(\alpha(P_{BP} + \rho g h))$$

P

$\Delta h$

$\Delta h$

$\Delta h$

$\Delta h$

EP 2 554 111 A1

*Fig.8*

EP 2 554 111 A1

*Fig.9*

## INTERNATIONAL SEARCH REPORT

| International application No. |
|---|
| PCT/JP2011/055226 |

**A. CLASSIFICATION OF SUBJECT MATTER**
*A61B5/02*(2006.01)i

According to International Patent Classification (IPC) or to both national classification and IPC

**B. FIELDS SEARCHED**

Minimum documentation searched (classification system followed by classification symbols)
A61B5/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2009-136656 A  (National Yang-Ming University), 25 September 2009 (25.09.2009), entire text; all drawings & US 2009/0149763 A1    & EP 2070472 A1 | 1-9 |
| A | JP 7-327940 A  (Nihon Kohden Corp.), 19 December 1995 (19.12.1995), entire text; all drawings & US 5649543 A | 1-9 |

☐ Further documents are listed in the continuation of Box C. ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 31 May, 2011 (31.05.11) | 07 June, 2011 (07.06.11) |

| Name and mailing address of the ISA/<br>Japanese Patent Office | Authorized officer |
|---|---|
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004313468 A

- JP 2003010139 A

**Non-patent literature cited in the description**

- **MCCOMBIE, DEVIN.** Development of a wearable blood pressure monitor using adaptive calibration of peripheral pulse transit time measurements. *Ph.D. Thesis,* 2008